# EUROPEAN PATENT SPECIFICATION

(11) **EP 4 329 709 B1**
(45) Date of publication and mention of the grant of the patent: **28.01.2026**
(21) Application number: 22723445.7
(22) Date of filing: 20.04.2022
(51) Int. Cl.: A61K 8/19, A61K 8/34, A61K 8/37, A61K 8/39, A61K 8/43, A61K 8/64, A61K 8/86, A61K 8/90, A61K 8/92, A61Q 5/04, A61Q 17/04

(54) **COMPOSITION COMPRISING DESFERRIOXAMINES AND UV LIGHT FILTER**
DESFERRIOXAMINE UND ZUSAMMENSETZUNG ENTHALTEND UV-LICHTSCHUTZFILTER
COMPOSITION CONTENANT DE LA DÉFÉROXAMINE ET UN FILTRE DE PROTECTION CONTRE LA LUMIÈRE UV

(30) Priority: 27.04.2021 EP 21170621
(43) Date of publication of application: 06.03.2024
(73) Proprietor: Evonik Operations GmbH, 45128 Essen (DE)
(72) Inventor: THIEMANN, Alexander, 25474 Hasloh (DE); STREHLOW, Sarah Alena, 22941 Bargteheide (DE); BRANDT, Kathrin Daniela, 40476 Düsseldorf (DE); KINDER, Michael, 25792 Neuenkirchen (DE); SCHMITT, Ellen, 22145 Hamburg (DE); STEINMETZER, Sabine, 65207 Wiesbaden (DE)
(74) Representative: Evonik Patent Association
(86) International application number: PCT/EP2022/060388
(87) International publication number: WO 2022/228963

(56) References cited:
- DE-A1- 10 050 155
- DE-A1- 102014 207 935

## Description

### Field of the invention

The invention relates to desferrioxamines and compositions containing UV light protection filters.

### Prior art

Desferrioxamines form a substance category within the iron-binding siderophores. A review is found, for example, in Hider and Kong, Chemistry and Biology of Siderophores, Natural Product Reports (2010), 27(5), 637-657.

Desferrioxamine B, also known as deferoxamine, is described for application in cosmetic applications. Thus, DE10050155 discloses cosmetic and dermatological preparations with a content of desferrioxamine B.

The need for sunscreen formulations with a high sun protection factor (SPF) has constantly increased in recent years. Formulations exhibit constantly increasing concentrations of UV light protection filters, in order to satisfy the demand for formulations with very high SPFs. Unfortunately, soiling by such sunscreen formulations of textiles can often be difficult to remove, since UV light protection filters exhibit a high affinity for the fibres.

EP 3 228 299 discloses, for solving this problem, a process for making it easier for cosmetic preparations containing UV light protection filters to be washed out from textiles, characterized in that 4-hydroxyacetophenone is added to the cosmetic.

DE102014207935 also addresses the problem of sunscreen formulations having a reduced tendency to stain textiles.

It is an object of the present invention to make available compositions containing UV light protection filters which make it possible to more effectively wash out UV light protection filters from textiles.

### Description of the invention

It has been found, surprisingly, that the compositions described subsequently, containing at least one desferrioxamine and/or at least one acid addition product of a desferrioxamine or salts thereof, are able to achieve the object addressed by the invention.

The present invention accordingly relates to compositions containing at least one desferrioxamine and/or at least one acid addition product of a desferrioxamine or salts thereof and also an organic UV light protection filter substance according to Claim 1.

The present invention additionally relates to a method for decreasing discolourations of a textile caused by a sunscreen formulation according to Claim 9.

The present invention additionally relates to the use of at least one desferrioxamine and/or at least one acid addition product of a desferrioxamine or salts thereof, in particular desferrioxamine B, for decreasing discolourations of a textile according to Claim 9.

The present invention additionally relates to the use of at least one desferrioxamine and/or at least one acid addition product of a desferrioxamine or salts thereof for more effectively washing out an organic UV light protection filter substance according to Claim 10.

An advantage of the present invention is that desferrioxamine and/or acid addition products of the desferrioxamine or salts thereof are better at keeping organic UV light protection filter substances ir solution.

An additional advantage of the present invention is that the compositions according to the invention are characterized by a relatively low oily and satisfactorily absorbing feeling on the skin.

An additional advantage of the present invention is that the compositions according to the invention exhibit an excellent shelf life with regard to the emulsion stability and accordingly can be kept for a long time.

An additional advantage of the present invention is that the compositions according to the invention have good spreadability and thus achieve a homogeneous distribution of the sunscreen factors on the skin.

An additional advantage of the present invention is that the compositions according to the invention have a particularly insignificant influence on the viscosity of a formulation and can be used over a broad pH range.

An additional advantage of the present invention is that the compositions according to the invention can be incorporated in a particularly easy and stable manner in cosmetic formulations.

An additional advantage of the present invention is that the compositions according to the invention exhibit particularly good skin sensorics and contribute to a reduction in the tackiness of sunscreen filters and reduce the whitening of the skin.

An additional advantage of the present invention is that the compositions according to the invention contribute to an improvement in the thermal stability of the formulation.

An additional advantage of the present invention is that the compositions according to the invention impart an excellent colour stability in the formulation.

An additional advantage of the present invention is that the compositions according to the invention exhibit improved shelf lives with regard to growth of certain microorganisms and accordingly can be kept for a long time.

Unless otherwise indicated, all stated percentages (%) are percentages by weight.

The present invention accordingly relates to a composition comprising
A) at least one substance chosen from desferrioxamines and acid addition products of a desferrioxamine or salts thereof, in particular desferrioxamine B,
B) at least one UV light protection filter substance,

characterized in that component B) is present in an amount of 6.0% by weight to 60% by weight, preferably from 8.0% by weight to 40% by weight, particularly preferably from 10.0% by weight to 30% by weight, the percentages by weight being with reference to the total composition,
characterized in that component B) comprises a UV light protection filter substance from the group of organic UV light protection filter substances.

Any desferrioxamine and acid addition product of a desferrioxamine or salt thereof, and also mixtures thereof, can be present according to the invention in component A).

Preferred desferrioxamines in this connection are chosen from desferrioxamine A_{1A}, desferrioxamine A_{1B}, desferrioxamine A₂, desferrioxamine B, desferrioxamine D₁, desferrioxamine D₂, desferrioxamine E, desferrioxamine Et₁, desferrioxamine Et₂, desferrioxamine Et₃, desferrioxamine G₁, desferrioxamine G_{2A}, desferrioxamine G_{2B}, desferrioxamine G_{2C}, desferrioxamine H, desferrioxamine N, desferrioxamine P₁, desferrioxamine T₁, desferrioxamine T₂, desferrioxamine T₃, desferrioxamine T₇, desferrioxamine T₈, desferrioxamine Te₁, desferrioxamine Te₂, desferrioxamine Te₃, desferrioxamine X₁, desferrioxamine X₂, desferrioxamine X₃ and desferrioxamine X₄, preferably desferrioxamine B, desferrioxamine D₁, desferrioxamine D₂, desferrioxamine E and desferrioxamine H, particularly preferably desferrioxamine B and desferrioxamine E.

For the nomenclature, compare if necessary Hider and Kong, Chemistry and Biology of Siderophores, Natural Product Reports (2010), 27(5), 637-657.

It is preferred according to the invention for the acid addition product of the desferrioxamine or salts thereof to be chosen from acid addition products of the acids chosen from formic acid, aminooxyacetic acid, amino acids, anthraquinonecarboxylic acid, succinic acid, aqueous hydrochloric acid, acetic acid, folic acid, glutaric acid, hydroxamic acid, malonic acid, methanesulfonic acid, nalidixic acid, N-(methylamino)benzoic acid, phenylsuccinic acid, phenylglutaric acid, phosphorous acid, phosphoric acid, hydrogen chloride, sulfurous acid, sulfuric acid, tartaric acid and citric acid, hydrogen chloride, methanesulfonic acid, acetic acid and amino acids being particularly preferred.

A preferred composition according to the invention is characterized in that component A) is present in an amount of 0.005% by weight to 10.0% by weight, preferably from 0.05% by weight to 5.0% by weight, particularly preferably from 0.1% by weight to 1.0% by weight, the percentages by weight being with respect to the total composition.

It is preferred according to the invention for component B) of the composition according to the invention to comprise at least two, preferably at least three, particularly preferably at least four, UV light protection filter substances.

Organic substances which are capable of absorbing ultraviolet radiation and re-emitting the absorbed energy in the form of longer-wave radiation, e.g. heat, for example can be present as UV light protection filter substances in component B).

UVB filters can be oil-soluble or water-soluble. Mention may be made, as oil-soluble UVB light protection filters, e.g., of:
3-benzylidenecamphor derivatives, e.g. 3-(4-methylbenzylidene)camphor (INCI: 4-Methylbenzylidene Camphor), 4-aminobenzoic acid derivatives, such as, e.g., 2-ethylhexyl 4-(dimethylamino)benzoate (INCI: Ethylhexyl Dimethyl PABA), 2-hydroxyethyl 4-{bis[2-(2-hydroxyethoxy)ethyl]amino}benzoate (INCI: PEG-25 PABA), cinnamic acid esters, such as, e.g., 2-ethylhexyl 4-methoxycinnamate (INCI: Ethylhexyl Methoxycinnamate), isopentyl 4-methoxycinnamate (INCI: Isoamyl p-Methoxycinnamate), 2-ethylhexyl 2-cyano-3,3-diphenylacrylate (INCI: Octocrylene), salicylic acid esters, such as, e.g., 2-ethylhexyl salicylate (INCI: Ethylhexyl Salicylate), homomenthyl salicylate (INCI: Menthyl Salicylate), 3,3,5-trimethylcyclohexyl salicylate (INCI: Homosalate), 2,2',2"-nitrilotriethanol 2-hydroxybenzoate (1:1) (INCI: TEA-Salicylate), benzophenone derivatives, such as, e.g., 2-hydroxy-4-methoxybenzophenone (INCI: Benzophenone-3), 2,2'-dihydroxy-4-methoxybenzophenone (INCI: Benzophenone-8), 2-hydroxy-4-methoxy-4'-methylbenzophenone (INCI: Benzophenone-10), triazine derivatives, such as, e.g., tris(2-ethylhexyl) 4,4',4"-(1,3,5-triazine-2,4,6-triyltriimino)tribenzoate (INCI: Ethylhexyl Triazone, obtainable, e.g., under the trade name Uvinul T 150 from BASF), iscotrizinol (INCI: Diethylhexyl Butamido Triazone) and 2,4,6-tri(4-biphenylyl)-1,3,5-triazine (INCI: Tris-Biphenyl Triazine).

An additional UVB filter is the (3-(4-(2,2-bis(ethoxycarbonyl)vinyl)phenoxy)propenyl)methoxysiloxane/dimethylsiloxane copolymer (INCI: Polysilicone-15), which can be obtained, for example, under the trade name Parsol SLX. Possible water-soluble UVB light protection filters are, for example:
salts of 2-phenylbenzimidazole-5-sulfonic acid, such as its alkali metal, alkaline earth metal, ammonium, alkylammonium, alkanolammonium and glucammonium salts, and also the sulfonic acid itself with the INCI name Phenylbenzimidazole Sulfonic Acid, sulfonic acid derivatives of benzophenone, such as, e.g., 5-benzoyl-4-hydroxy-2-methoxybenzenesulfonic acid (INCI: Benzophenone-4) and its salts, benzensulfonic acid derivatives of 3-benzylidenecamphor, such as, e.g., 4-[(E)-(4,7,7-trimethyl-3-oxobicyclo[2.2.1]hept-2-ylidene)methyl]benzenesulfonic acid (INCI: Benzylidene Camphor Sulfonic Acid) and its salts.

Possible typical oil-soluble UVA/broadband light protection filters are, for example, benzoylmethane derivatives, such as, for example, 1-(4-methoxyphenyl)-3-[4-(2-methyl-2-propanyl)phenyl]-1,3-propanedione (INCI: Butyl Methoxydibenzoylmethane) or 1-(4-isopropylphenyl)-3-phenyl-1,3-propanedione (INCI: Isopropyl Dibenzoylmethane), triazine derivatives, such as, e.g., 2,2'-[6-(4-methoxyphenyl)-1,3,5-triazine-2,4-diyl]bis{5-[(2-ethylhexyl)oxy]phenol} (INCI: Bis-Ethylhexyloxyphenol Methoxyphenyl Triazine, obtainable, e.g., under the trade name Tinosorb S from BASF), N,N'-bis[4-[5-(1,1-dimethylpropyl)-2-benzoxazolyl]phenyl]-N"-(2-ethylhexyl)-1,3,5-triazine-2,4,6-triamine (INCI: Ethylhexyl Bis-Isopentylbenzoxazolylphenyl Melamine, obtainable under the trade name Uvasorb K2A from 3V Sigma) and benzophenone derivatives, such as, e.g., hexyl 2-(4-diethylamino-2-hydroxybenzoyl)benzoate (INCI: Diethylamino Hydroxybenzoyl Hexyl Benzoate).

Possible water-soluble UVA/broadband light protection filters are, for example: 3,3'-(1,4-phenylenedimethylene)bis(7,7-dimethyl-2-oxobicyclo[2.2.1]hept-1-ylmethanesulfonic acid) and the salts thereof, in particular the corresponding sodium, potassium or triethanolammonium salt, which is also described as benzene-1,4-di(2-oxo-3-bornylidenemethyl-10-sulfonic acid) and has the INCI description Terephthalylidene Dicamphor Sulfonic Acid (obtainable under the trade name Mexoryl SX), 2,2'-(1,4-phenylene)bis(6-sulfo-1H-benzimidazole-4-sulfonic acid) and its salts, the corresponding sodium, potassium or triethanolammonium salts, e.g. disodium 2,2'-(1,4-phenylene)bis(6-sulfo-1H-benzimidazol-4-sulfonate) with the INCI description Disodium Phenyl Dibenzimidazole Tetrasulfonate, trade name e.g. Neo Heliopan AP.

Additional UVA/broadband filters are, e.g. 2,2'-methylenebis[6-(2H-benzotriazol-2-yl)-4-(2,4,4-trimethyl-2-pentanyl)phenol] (INCI: Methylene Bis-Benzotriazolyl Tetramethylbutylphenol, obtainable, e.g., under the trade name Tinosorb M from BASF), 2-(2H-benzotriazol-2-yl)-4-methyl-6-[2-methyl-3-[1,3,3,3-tetramethyl-1-[(trimethylsilyl)oxy]disiloxanyl]propyl]phenol (INCI: Drometrizole Trisiloxane, trade name: Mexoryl XL), (1R,2S,5R)-2-isopropyl-5-methylcyclohexyl 2-aminobenzoate (INCI: Menthyl Anthranilate) and 2-ethoxyethyl (2E)-3-(4-methoxyphenyl)acrylate (INCI: Cinoxate).

The UV filters can also obviously be present in mixtures in the compositions according to the invention.

In addition to the soluble substances mentioned, also possible are micronized organic pigments, such as, for example, 2,2'-methylenebis[6-(2H-benzotriazol-2-yl)-4-(2,4,4-trimethyl-2-pentanyl)phenol] (INCI: Methylene Bis-Benzotriazolyl Tetramethylbutylphenol, obtainable, e.g., under the trade name Tinosorb M from BASF) with a particle size of < 200 nm, which can be obtained, e.g., as a 50% aqueous dispersion.

Furthermore, additional suitable UV light protection filters can be taken from the review by P. Finkel in SOFW Journal, 122, 543 (1996), or from the chapter, "The Chemistry of Ultraviolet Filters", by N.A. Shaath in "Principles and Practice of Photoprotection", S.Q. Wang and H.W. Lim (eds.), Springer International Publishing, Switzerland, 2016.

It is preferred according to the invention for the component B) not to contain a UV light protection filter substance from the group octyl methoxycinnamate, methylbenzylidenecamphor and butyl methoxydibenzoylmethane.

It is preferred according to the invention for the component B) to comprise a UV light protection filter substance from the group of the triazine derivatives.

A preferred composition according to the invention is characterized in that component B) comprises a UV light protection filter substance from the group,
2,2'-[6-(4-methoxyphenyl)-1,3,5-triazine-2,4-diyl]bis{5-[(2-ethylhexyl)oxy]phenol} (INCI: Bis-Ethylhexyloxyphenol Methoxyphenyl Triazine),
2-ethylhexyl salicylate (INCI: Ethylhexyl Salicylate),
2,4,6-trianilino(p-carbo-2-ethylhexyl-1-oxy)-1,3,5-triazine (INCI: Ethylhexyl Triazone),
hexyl 2-(4-diethylamino-2-hydroxybenzoyl)benzoate (INCI: Diethylamino Hydroxybenzoyl Hexyl Benzoate) and
1-(4-methoxyphenyl)-3-[4-(2-methyl-2-propanyl)phenyl]-1,3-propanedione (INCI: Butyl Methoxydibenzoylmethane).

The compositions according to the invention can, e.g., comprise at least one further additional component chosen from the group of the
emollients, emulsifiers, coemulsifiers, thickeners/viscosity regulators/stabilizers, antioxidants, hydrotropes (or polyols), solids and fillers, pearlescent additives, insect repellents, self-tanning agents, preservatives, conditioners, perfumes, colourants, cosmetic active substances, care additives, superfatting agents and solvents.

Substances which can be used as exemplary representatives of the individual groups are known to a person skilled in the art and can, for example, be taken from German Application DE 102008001788.4.

As regards further optional components and also the amounts used of these components, reference is expressly made to the relevant handbooks known to a person skilled in the art, e.g. K. Schrader, "Grundlagen und Rezepturen der Kosmetika [Fundamentals and Formulations of Cosmetics]", 2nd edition, pages 329 to 341, Hüthig Buch Verlag, Heidelberg.

The amounts of the respective additives depend on the intended use.

Typical starting formulations for the relevant applications are known prior art and are contained for example in the brochures of the manufacturers of the relevant base materials and active substances. These existing formulations can generally be adopted unchanged. However, if necessary, for adjustment and optimization, the desired modifications can be executed in a straightforward manner through simple tests.

Compositions according to the invention can, for example, be used as skin care, face care, head care, body care, intimate care, foot care, hair care, nail care, dental care or oral care products.

Compositions according to the invention can, for example, be used in the form of an emulsion, a suspension, a solution, a cream, a salve, a paste, a gel, an oil, a powder, an aerosol, a stick, a spray, a cleansing product, a make-up or sunscreen preparation or a face lotion.

Preferred compositions according to the invention are emulsions, in particular chosen from oil-in-water emulsions and water-in-oil emulsions, oil-in-water emulsions being particularly preferred.

The present invention additionally relates to a process for decreasing discolourations of a textile caused by a sunscreen formulation comprising the process stage
Making available the sunscreen formulation in the form of a composition containing
A) at least one substance chosen from desferrioxamines and acid addition products of a desferrioxamine or salts thereof, in particular desferrioxamine B,
B) at least one UV light protection filter substance from the group of organic UV light protection filter substances, and/or
in the form of a composition according to the invention.

According to the invention, the method according to the invention preferably comprises in addition the process stage
Washing the textile with an aqueous composition, preferably an aqueous surfactant composition.

The compositions mentioned above as preferred in connection with the compositions according to the invention are preferably used according to their preference in the method according to the invention.

The present invention additionally relates to the use of at least one substance chosen from desferrioxamines and acid addition products of a desferrioxamine or salts thereof, in particular desferrioxamine B, for improving the ability to wash out at least one organic UV light protection filter substance from a textile.

In the uses according to the invention, the components mentioned above in connection with the compositions according to the invention as components preferably present are preferably used.

The present invention is described by way of example in the examples cited below, without the invention, the scope of application of which results from the entirety of the description and from the claims, being restricted to the embodiments mentioned in the examples.

The following figures are an integral part of the examples:
Figure 1: Results of colorimetric measurement
Figure 2: Results of colorimetric measurement; 4-hydroxyacetophenone comparison

### Examples:

### Example 1:

### For the comparison of the ability of marks of light protection filter to be washed out from textiles, different cosmetic compositions

(Formulations 1-6) were prepared and 0.6 g was applied evenly in circular fashion (diameter d = 45 mm) to a cotton fabric. Subsequently, the fabric was dried under laboratory conditions at 40°C for 1 h in a drying cupboard and subsequently covered with baking paper, and ironed with an iron at level 2 for 1 min. Subsequently, the colour intensity of the marks was measured (first measurement) by means of a colorimeter (NR145, 3nh).

The colorimeter measurement was used for the recording or the comparison of the discolouration before and after the washing of the textiles. The principle of the colour measurement is based on an illumination of the sample and on the recording of the reflectance spectrum and on the direct conversion into colorimetric data. The data determined describe the colour of a sample in the L*a*b* colour space (also: CIELAB; DIN EN ISO 11664-4). In this connection, the a coordinate gives the colour intensity and chromaticity between green and red, the b coordinate gives the colour intensity and the chromaticity between blue and yellow and the L coordinate gives the lightness. For the comparison, the ΔE value between the discolourations and the untreated fabric was determined before and after washing and used as a measure of the Euclidean colour difference to each other.

Subsequent to the first measurement, the fabric was washed at 60°C (1 h; 60°C; Ariel Colour powder), and subsequently again without detergent (1 h; 60°C; water). After washing, the fabric was dried in air under laboratory conditions and the colour intensity of the marks was measured again (second measurement) by means of a colorimeter (NR145, 3nh).

The formulation constituents in the following compositions (Formulations 1-6) are referred to in the form of the generally accepted INCI nomenclature with use of the English terms. All concentrations in the use examples are, unless otherwise indicated, given as % by weight.

### Formulations 1-6: Sunscreen creams

In total, 3x6 O/W emulsions (sunscreen creams) were prepared.

Formulation 1 comprised no UV filter (neg. control) and Formulations 2-6 contained five UV filters in different combinations. Each formulation was prepared each time without complexing agent, with EDTA and with desferrioxamine. The pH of all formulations was adjusted with citric acid to 5.5.

| **Raw material** | **Formulati on 1** | | | **Formul ation 2** | | | **Formulat ion 3** | | | **Formulat ion 4** | | | **Formulat ion 5** | | | **Formulat ion 6** | | |
|---|---|---|---|---|---|---|---|---|---|---|---|---|---|---|---|---|---|---|
| Desferrioxamine [mmol] | - | - | 2 .7 | - | - | 2 .7 | - | - | 2 .7 | - | - | 2 .7 | - | - | 2 .7 | - | - | 2 .7 |
| Disodium EDTA [mmol] | - | 2 .7 | - | - | 2 .7 | - | - | 2 .7 | - | - | 2 .7 | - | - | 2 .7 | - | - | 2 .7 | - |
| Glycerin | 3.00 | | | 3.00 | | | 3.00 | | | 3.00 | | | 3.00 | | | 3.00 | | |
| Caprylyl Glycol; Glyceryl Caprylate; Glycerin; Phenylpropanol; Aqua | 1.00 | | | 1.00 | | | 1.00 | | | 1.00 | | | 1.00 | | | 1.00 | | |
| Magnesium Aluminium Silicate | 0.70 | | | 0.70 | | | 0.70 | | | 0.70 | | | 0.70 | | | 0.70 | | |
| Xanthan Gum | 0.30 | | | 0.30 | | | 0.30 | | | 0.30 | | | 0.30 | | | 0.30 | | |
| Glyceryl Stearate Citrate; Cetearyl Alcohol; Glyceryl Caprylate | 6.00 | | | 6.00 | | | 6.00 | | | 6.00 | | | 6.00 | | | 6.00 | | |
| Isoamyl Laurate | 3.00 | | | 3.00 | | | 3.00 | | | 3.00 | | | 3.00 | | | 3.00 | | |
| Phenoxyethyl Caprylate | 15.00 | | | 15.00 | | | 15.00 | | | 15.00 | | | 15.00 | | | 15.00 | | |
| Dimethicone | 0.30 | | | 0.30 | | | 0.30 | | | 0.30 | | | 0.30 | | | 0.30 | | |
| Tocopherol; Helianthus Annuus Seed Oil | 0.05 | | | 0.05 | | | 0.05 | | | 0.05 | | | 0.05 | | | 0.05 | | |
| Ethylhexyl Salicylate | - | | | 5.00 | | | 5.00 | | | - | | | | | | - | | |
| Bis-Ethylhexyloxyphenol Methoxyphenyl Triazine | - | | | 2.00 | | | - | | | 5.00 | | | | | | - | | |
| Butyl Methoxydibenzoylme thane | - | | | 4.00 | | | - | | | - | | | 5.00 | | | | | |
| Ethylhexyl Triazone | - | | | 1.00 | | | - | | | - | | | | | | - | | |
| Diethylamino Hydroxybenzoyl Hexyl Benzoate | - | | | - | | | - | | | - | | | | | | 7.00 | | |
| Aqua | to 100 | | | to 100 | | | to 100 | | | to 100 | | | to 100 | | | to 100 | | |

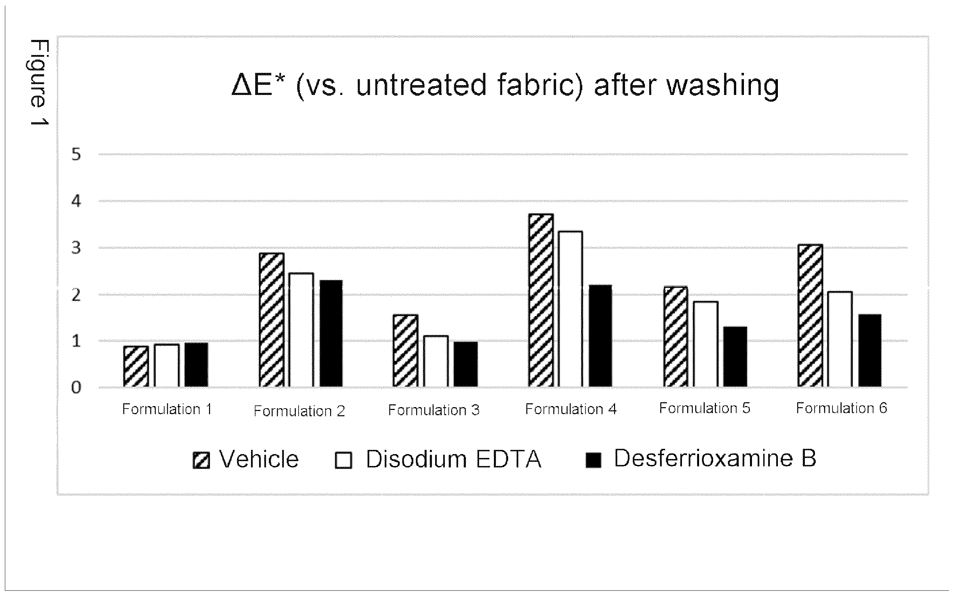

The results (Figure 1) show that, with Formulations 2-6 according to the invention containing desferrioxamine, in comparison with Formulation 1, more efficient washing is surprisingly obtained and that, surprisingly, the effectiveness turned out to be significantly higher than the effectiveness through the functionally similar comparative substance Disodium EDTA.

### Example 2:

Following EP 3 228 299 (Example Formulation 2 from EP 3 228 299), cosmetic compositions (O/W sunscreen creams) were prepared with 4-hydroxyacetophenone (Formulation 7) and desferrioxamine B (Formulation 8) and both together (Formulation 9), and the ability to wash out light protection filter marks from textiles was investigated according to the methodology described above.

The formulation constituents in the following compositions (Formulation 7-9) are referred to in the form of the generally accepted INCI nomenclature. All concentrations in the use examples are, unless otherwise indicated, given as % by weight.

### Formulations 7-9: Sunscreen creams

In total, 3 O/W emulsions (sunscreen creams) were prepared with the 6 UV filters Homosalate, Ethylhexyl Salicylate, Bis-Ethylhexyloxyphenol Methoxyphenyl Triazine, Ethylhexyl Triazone, Butyl Methoxydibenzoylmethane and Phenylbenzimidazole Sulfonic Acid.

The formulation was each time prepared with 4-hydroxyacetophenone (Formulation 7), desferrioxamine (Formulation 8) and both raw materials (Formulation 9). The pH of all formulations was adjusted with NaOH to 7.3.

| **Raw material** | **Formulation 7** | **Formulation 8** | **Formulation 9** |
|---|---|---|---|
| Hydroxyacetophenone | 0.50 (2.70 mmol) | - | 0.50 (2.70 mmol) |
| Desferrioxamine | - | 0.21 (2.70 mmol) | 0.21 (2.70 mmol) |
| Sodium Carboxymethylcellulose | 0.50 | | |
| Tocopheryl Acetate | 0.06 | | |
| Ethylhexylglycerin | 0.50 | | |
| C12-15 Alkyl Benzoate | 3.00 | | |
| Butylene Glycol Dicaprylate/Dicaprate | 1.00 | | |
| C18-38 Alkyl Hydroxystearol Stearate | 1.50 | | |
| Glyceryl Stearate | 1.00 | | |
| Sodium Stearoyl Glutamate | 0.30 | | |
| Silica Dimethyl Silylate | 0.50 | | |
| Triacontanyl PVP | 1.00 | | |
| Glycerin | 2.00 | | |
| Sodium Hydroxide | q.s (pH 7.3) | | |
| Stearyl Alcohol | 1.00 | | |
| Acrylates/C10-30 Alkyl Acrylate Crosspolymer | 0.15 | | |
| Xanthan Gum | 0.40 | | |
| Denat. Alcohol | 5.00 | | |
| Tetrasodium Iminodisuccinate | 0.75 | | |
| Homosalate | 8.50 | | |
| Ethylhexyl Salicylate | 4.50 | | |
| Bis-Ethylhexyloxyphenol Methoxyphenyl Triazine | 2.00 | | |
| Ethylhexyl Triazone | 1.00 | | |
| Butyl Methoxydibenzoylmethane | 3.50 | | |
| Phenylbenzimidazole Sulfonic Acid | 1.00 | | |
| Aqua | to 100 | | |

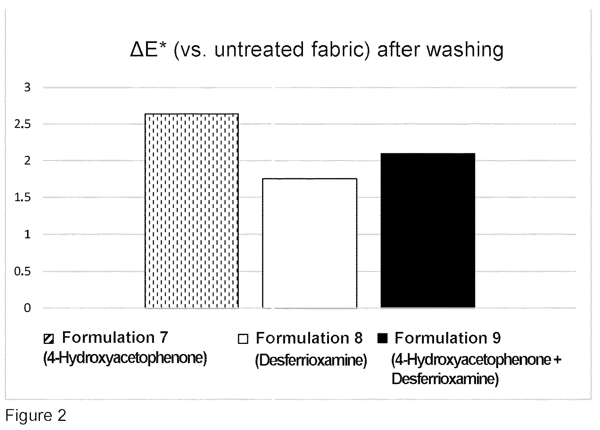

The results in Figure 2 show that, with Formulations 8 and 9 according to the invention, containing desferrioxamine, in comparison with Formulation 7, containing only 4-hydroxyacetophenone, more efficient washing is surprisingly obtained and consequently, surprisingly, the effectiveness turned out to be significantly higher than the effectiveness through the comparative substance 4-hydroxyacetophenone.

### Example 3:

Following Example 1 and 2, cosmetic compositions (O/W sunscreen creams) were prepared with 4-hydroxyacetophenone, Disodium EDTA and desferrioxamine (according to the invention) and without any of the forementioned raw materials. The ability to wash off anorganic UV light protection filter marks from textiles was investigated according to the methodology described in Example 1.

The formulation constituents in the following compositions (Formulation 10-13) are referred to in the form of the generally accepted INCI nomenclature.

All concentrations in the examples are, unless otherwise indicated, given as % by weight.

### Formulations 10-13: Sunscreen creams

In total, 4 O/W emulsions (sunscreen creams) were prepared with 2 insoluble, anorganic UV filter pigments, the metal oxides Titanium dioxide and Zinc Oxide.

The formulation was each time prepared with 4-hydroxyacetophenone (Formulation 11), Disodium EDTA (Formulation 12) and desferrioxamine B (Formulation 13) and without any of the forementioned raw materials (Formulation 10). The pH of all formulations was adjusted to 5.5.

| **Raw material** | **Formulation 10** | **Formulation 11** | **Formulation 12** | **Formulation 13** |
|---|---|---|---|---|
| Desferrioxamine [mmol] | - | 2.70 | | |
| Disodium EDTA [mmol] | - | | 2.70 | |
| Hydroxyacetophenone [mmol] | - | | | 2.70 |
| Glycerin | 3.00 | | | |
| Caprylyl Glycol; Glyceryl Caprylate; Glycerin; Phenylpropanol; Aqua | 1.00 | | | |
| Magnesium Aluminium Silicate | 0.70 | | | |
| Xanthan Gum | 0.30 | | | |
| Glyceryl Stearate Citrate; Cetearyl Alcohol; Glyceryl Caprylate | 6.00 | | | |
| Isoamyl Laurate | 3.00 | | | |
| Phenoxyethyl Caprylate | 15.00 | | | |
| Dimethicone | 0.30 | | | |
| Tocopherol; Helianthus Annuus Seed Oil | 0.05 | | | |
| Zinc oxide | 10.00 | | | |
| Titanium dioxide | 10.00 | | | |
| Aqua | to 100 | | | |

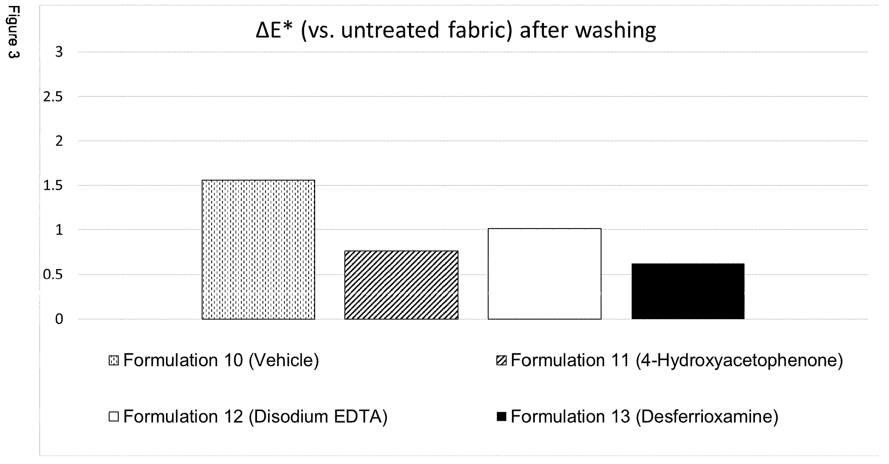

The results in Figure 3 show that with Formulation 13 according to the invention, containing desferrioxamine, in comparison to Formulation 11, containing only 4-hydroxyacetophenone, and formulation 12, containing only Disodium EDTA, more efficient washing off the marks caused by anorganic UV filter pigments is surprisingly obtained and consequently, surprisingly, the effectiveness turned out to be significantly higher than the effectiveness through the comparative substances 4-hydroxyacetophenone and Disodium EDTA.

### Example 4:

Sensory improvements of a formulation according to the invention (Formulation 11 from Example 3) in comparison to a formulation without desferrioxamine (Formulation 10 from Example 3) are observed on a likert scale from 1 (very bad) to 5 (very good) by a group of 4 trained test persons, The sensory test results are summarized in Table 1.

**Table 1: Sensory rating (average values)**

| | Formulation 10 | Formulation 11 |
|---|---|---|
| Skin whitening by UV filters | 1,25 | 2,25 |
| Skin Feeling after 3 min | 2,25 | 3,00 |
| Tackiness | 2 | 2.25 |
| Spreadability | 2,25 | 3,25 |

The sunscreen formulation in accordance to the invention (Formulation 11) improves the spreadability, tackiness and skin feeling and reduces the whitening effect of the formulation in comparison to the vehicle formulation (Formulation 10) without desferrioxamine.

## Claims

1. Composition comprising
A) at least one substance chosen from desferrioxamines and acid addition products of a desferrioxamine or salts thereof,
B) at least one UV light protection filter substance,
**characterized in that** component B) is present in an amount of 6.0% by weight to 60% by weight, preferably from 8.0% by weight to 40% by weight, particularly preferably from 10.0% by weight to 30% by weight, the percentages by weight being with respect to the total composition,
**characterized in that** component B) comprises a UV light protection filter substance from the group of organic UV light protection filter substances.

2. Composition according to Claim 1, **characterized in that** the desferrioxamine is chosen from desferrioxamine A_{1A}, desferrioxamine A_{1B}, desferrioxamine A₂, desferrioxamine B, desferrioxamine D₁, desferrioxamine D₂, desferrioxamine E, desferrioxamine Et₁, desferrioxamine Et₂, desferrioxamine Et₃, desferrioxamine G₁, desferrioxamine G_{2A}, desferrioxamine G_{2B}, desferrioxamine G_{2C}, desferrioxamine H, desferrioxamine N, desferrioxamine P₁, desferrioxamine T₁, desferrioxamine T₂, desferrioxamine T₃, desferrioxamine T₇, desferrioxamine T₈, desferrioxamine Te₁, desferrioxamine Te₂, desferrioxamine Te₃, desferrioxamine X₁, desferrioxamine X₂, desferrioxamine X₃ and desferrioxamine X₄, preferably desferrioxamine B, desferrioxamine D₁, desferrioxamine D₂, desferrioxamine E and desferrioxamine H, particularly preferably desferrioxamine B and desferrioxamine E.

3. Composition according to Claim 1 or 2, **characterized in that** the acid addition product of the desferrioxamine or salt thereof is chosen from acid addition products of the acids chosen from formic acid, aminooxyacetic acid, amino acids, anthraquinonecarboxylic acid, succinic acid, aqueous hydrochloric acid, acetic acid, folic acid, glutaric acid, hydroxamic acid, malonic acid, methanesulfonic acid, nalidixic acid, N-(methylamino)benzoic acid, phenylsuccinic acid, phenylglutaric acid, phosphorous acid, phosphoric acid, sulfurous acid, sulfuric acid, tartaric acid and citric acid.

4. Composition according to at least one of the preceding claims, **characterized in that** component A) is present in an amount of 0.005% by weight to 10.0% by weight, preferably from 0.05% by weight to 5.0% by weight, particularly preferably from 0.1% by weight to 1.0% by weight, the percentages by weight being with respect to the total composition.

5. Composition according to at least one of the preceding claims, **characterized in that** component B) comprises at least two, preferably at least three, particularly preferably at least four, UV light protection filter substances.

6. Composition according to at least one of the preceding claims, **characterized in that** component B) comprises a UV light protection filter substance from the group of the triazine derivatives.

7. Composition according to at least one of Claims 1 to 4, **characterized in that** component B) comprises a UV light protection filter substance from the group
2,2'-[6-(4-methoxyphenyl)-1,3,5-triazine-2,4-diyl]bis{5-[(2-ethylhexyl)oxy]phenol},
2-ethylhexyl salicylate,
2,4,6-trianilino(p-carbo-2-ethylhexyl-1-oxy)-1,3,5-triazine,
hexyl 2-(4-diethylamino-2-hydroxybenzoyl)benzoate and
1-(4-methoxyphenyl)-3-[4-(2-methyl-2-propanyl)phenyl]-1,3-propanedione.

8. Composition according to at least one of the preceding claims, **characterized in that** at least one further additional component is present, chosen from the group of the emollients, emulsifiers, coemulsifiers, thickeners/viscosity regulators/stabilizers, antioxidants, hydrotropes (or polyols), solids and fillers, pearlescent additives, insect repellents, self-tanning agents, preservatives, conditioners, perfumes, colourants, cosmetic active substances, care additives, superfatting agents and solvents.

9. Process for decreasing discolourations of a textile caused by a sunscreen formulation comprising the process stage
Making available the sunscreen formulation
in the form of a composition containing
A) at least one substance chosen from desferrioxamines and acid addition products of a desferrioxamine or salts thereof, in particular desferrioxamine B,
B) at least one UV light protection filter substance from the group of organic UV light protection filter substances, and/or
in the form of a composition according to at least one of the preceding claims.

10. Use of at least one substance chosen from desferrioxamines and acid addition products of a desferrioxamine or salts thereof, in particular desferrioxamine B, for improving the ability to wash out at least one organic UV light protection filter substance from a textile.

## Patentansprüche

1. Zusammensetzung, umfassend
A) mindestens eine Substanz ausgewählt aus Desferrioxaminen und Säureanlagerungsprodukten eines Desferrioxamins oder Salzen davon,
B) mindestens eine UV-Lichtschutzfiltersubstanz,
**dadurch gekennzeichnet, dass** Komponente B) in einer Menge von 6,0 Gew.-% bis 60 Gew.-%, bevorzugt von 8,0 Gew.-% bis 40 Gew.-%, besonders bevorzugt von 10,0 Gew.-% bis 30 Gew.-% vorliegt, wobei sich die Gewichtsprozente auf die Gesamtzusammensetzung beziehen,
**dadurch gekennzeichnet, dass** Komponente B) eine UV-Lichtschutzfiltersubstanz aus der Gruppe der organischen UV-Lichtschutzfiltersubstanzen umfasst.

2. Zusammensetzung nach Anspruch 1, **dadurch gekennzeichnet, dass** das Desferrioxamin ausgewählt ist aus Desferrioxamin A_{1A}, Desferrioxamin A_{1B}, Desferrioxamin A₂, Desferrioxamin B, Desferrioxamin D₁, Desferrioxamin D₂, Desferrioxamin E, Desferrioxamin Et₁, Desferrioxamin Et₂, Desferrioxamin Et₃, Desferrioxamin G₁, Desferrioxamin G_{2A}, Desferrioxamin G_{2B}, Desferrioxamin G_{2C}, Desferrioxamin H, Desferrioxamin N, Desferrioxamin P₁, Desferrioxamin T₁, Desferrioxamin T₂, Desferrioxamin T₃, Desferrioxamin T₇, Desferrioxamin T_{B}, Desferrioxamin Te₁, Desferrioxamin Te₂, Desferrioxamin Te₃, Desferrioxamin X₁, Desferrioxamin X₂, Desferrioxamin X₃ und Desferrioxamin X₄, bevorzugt Desferrioxamin B, Desferrioxamin D₁, Desferrioxamin D₂, Desferrioxamin E und Desferrioxamin H, besonders bevorzugt Desferrioxamin B und Desferrioxamin E.

3. Zusammensetzung nach Anspruch 1 oder 2, **dadurch gekennzeichnet, dass** das Säureanlagerungsprodukt des Desferrioxamins oder Salz davon ausgewählt ist aus Säureanlagerungsprodukten der Säuren ausgewählt aus Ameisensäure, Aminooxyessigsäure, Aminosäuren, Anthrachinoncarbonsäure, Bernsteinsäure, Chlorwasserstoffsäure, Essigsäure, Folsäure, Glutarsäure, Hydroxamsäure, Malonsäure, Methansulfonsäure, Nalidixinsäure, N-(Methylamino)benzoesäure, Phenylbernsteinsäure, Phenylglutarsäure, phosphoriger Säure, Phosphorsäure, schwefliger Säure, Schwefelsäure, Weinsäure und Citronensäure.

4. Zusammensetzung nach mindestens einem der vorhergehenden Ansprüche, **dadurch gekennzeichnet, dass** Komponente A) in einer Menge von 0,005 Gew.-% bis 10,0 Gew.-%, bevorzugt von 0,05 Gew.-% bis 5,0 Gew.-%, besonders bevorzugt von 0,1 Gew.-% bis 1,0 Gew.-% vorliegt, wobei sich die Gewichtsprozente auf die Gesamtzusammensetzung beziehen.

5. Zusammensetzung nach mindestens einem der vorhergehenden Ansprüche, **dadurch gekennzeichnet, dass** Komponente B) mindestens zwei, bevorzugt mindestens drei, besonders bevorzugt mindestens vier, UV-Lichtschutzfiltersubstanzen umfasst.

6. Zusammensetzung nach mindestens einem der vorhergehenden Ansprüche, **dadurch gekennzeichnet, dass** Komponente B) eine UV-Lichtschutzfiltersubstanz aus der Gruppe der Triazinderivate umfasst.

7. Zusammensetzung nach mindestens einem der Ansprüche 1 bis 4, **dadurch gekennzeichnet, dass** Komponente B) eine UV-Lichtschutzfiltersubstanz aus der Gruppe 2,2'-[6-(4-Methoxyphenyl)-1,3,5-triazin-2,4-diyl]bis{5-[(2-ethylhexyl)oxy]phenol}, Salicylsäure-2-ethylhexylester, 2,4,6-Trianilino(p-carbo-2-ethylhexyl-1-oxy)-1,3,5-triazin, 2-(4-Diethylamino-2-hydroxybenzoyl)benzoesäurehexylester und 1-(4-Methoxyphenyl)-3-[4-(2-methyl-2-propanyl)phenyl]-1,3-propandion umfasst.

8. Zusammensetzung nach mindestens einem der vorhergehenden Ansprüche, **dadurch gekennzeichnet, dass** mindestens eine weitere zusätzliche Komponente enthalten ist, ausgewählt aus der Gruppe der Emollienzien, Emulgatoren, Coemulgatoren, Verdickungsmittel/Viskositätsregler/Stabilisatoren, Antioxidantien, Hydrotrope (oder Polyole), Fest- und Füllstoffe, Perlglanzadditive, Insektenrepellentien, Selbstbräuner, Konservierungsstoffe, Konditioniermittel, Parfüme, Farbstoffe, kosmetischen Wirkstoffe, Pflegeadditive, Überfettungsmittel und Lösungsmittel.

9. Verfahren zum Vermindern von durch eine Sonnenschutzformulierung hervorgerufenen Verfärbungen eines Textils, umfassend den Verfahrensschritt
Bereitstellen der Sonnenschutzformulierung in Form einer Zusammensetzung, enthaltend
A) mindestens eine Substanz ausgewählt aus Desferrioxaminen und Säureanlagerungsprodukten eines Desferrioxamins oder Salzen davon, insbesondere Desferrioxamin B,
B) mindestens eine UV-Lichtschutzfiltersubstanz aus der Gruppe der organischen UV-Lichtschutzfiltersubstanzen, und/oder
in Form einer Zusammensetzung nach mindestens einem der vorhergehenden Ansprüche.

10. Verwendung mindestens einer Substanz ausgewählt aus Desferrioxaminen und Säureanlagerungsprodukten eines Desferrioxamins oder Salzen davon, insbesondere Desferrioxamin B, zur Verbesserung der Auswaschbarkeit mindestens einer organischen UV-Lichtschutzfiltersubstanz aus einem Textil.

## Revendications

1. Composition comprenant
A) au moins une substance choisie parmi les desferrioxamines et les produits d'additions d'acide d'une desferrioxamine ou leurs sels,
B) au moins une substance de filtre de protection contre de la lumière UV,
**caractérisée en ce que** le composant B) est présent en une quantité de 6,0 % en poids à 60 % en poids, de préférence de 8,0 % en poids à 40 % en poids, particulièrement préférablement de 10,0 % en poids à 30 % en poids, les pourcentages en poids étant par rapport à la composition totale,
**caractérisée en ce que** le composant B) comprend une substance de filtre de protection contre de la lumière UV du groupe des substances de filtre de protection contre de la lumière UV organiques.

2. Composition selon la revendication 1, **caractérisée en ce que** la desferrioxamine est choisie parmi la desferrioxamine A_{1A}, desferrioxamine A_{1B}, desferrioxamine A₂, desferrioxamine B, desferrioxamine D₁, desferrioxamine D₂, desferrioxamine E, desferrioxamine Et₁, desferrioxamine Et₂, desferrioxamine Et₃, desferrioxamine G₁, desferrioxamine G_{2A}, desferrioxamine G_{2B}, desferrioxamine G_{2C}, desferrioxamine H, desferrioxamine N, desferrioxamine P₁, desferrioxamine T₁, desferrioxamine T₂, desferrioxamine T₃, desferrioxamine T₇, desferrioxamine T₈, desferrioxamine Te₁, desferrioxamine Te₂, desferrioxamine Te₃, desferrioxamine X₁, desferrioxamine X₂, desferrioxamine X₃ et desferrioxamine X₄, de préférence la desferrioxamine B, desferrioxamine D₁, desferrioxamine D₂, desferrioxamine E et desferrioxamine H, particulièrement préférablement la desferrioxamine B et desferrioxamine E.

3. Composition selon la revendication 1 ou 2, **caractérisée en ce que** le produit d'addition acide de la desferrioxamine ou leur sel est choisi parmi les produits d'addition acide des acides choisis parmi l'acide formique, l'acide aminooxyacétique, les acides aminés, l'acide anthraquinonecarboxylique, l'acide succinique, l'acide chlorhydrique aqueux, l'acide acétique, l'acide folique, l'acide glutarique, l'acide hydroxamique, l'acide malonique, l'acide méthanesulfonique, l'acide nalidixique, l'acide N-(méthylamino)benzoïque, l'acide phénylsuccinique, l'acide phénylglutarique, l'acide phosphoreux, l'acide phosphorique, l'acide sulfureux, l'acide sulfurique, l'acide tartrique et l'acide citrique.

4. Composition selon au moins une des revendications précédentes, **caractérisée en ce que** le composant A) est présent en une quantité de 0,005 % en poids à 10,0 % en poids, de préférence de 0,05 % en poids à 5,0 % en poids, particulièrement préférablement de 0,1 % en poids à 1,0 % en poids, les pourcentages en poids étant par rapport à la composition totale.

5. Composition selon au moins l'une quelconque des revendications précédentes, **caractérisée en ce que** le composant B) comprend au moins deux, de préférence au moins trois, particulièrement préférablement au moins quatre substances de filtre de protection contre de la lumière UV.

6. Composition selon au moins l'une des revendications précédentes, **caractérisée en ce que** le composant B) comprend une substance de filtre de protection contre de la lumière UV du groupe des dérivés de triazine.

7. Composition selon au moins l'une quelconque des revendications 1 à 4, **caractérisée en ce que** le composant B) comprend une substance de filtre de protection contre de la lumière UV du groupe 2,2'-[6-(4-méthoxyphényl)-1,3,5-triazine-2,4-diyl]bis{5-[(2-éthylhexyl)oxy]phénol}, salicylate de 2-éthylhexyle, 2,4,6-trianilino(p-carbo-2-éthylhexyl-1-oxy)-1,3,5-triazine, 2-(4-diéthylamino-2-hydroxybenzoyl)benzoate d'hexyle et 1-(4-méthoxyphényl)-3-[4-(2-méthyl-2-propanyl)phényl]-1,3-propanedione.

8. Composition selon au moins l'une des revendications précédentes, **caractérisée en ce qu'**au moins un autre composant est présent, choisi dans le groupe des émollients, des émulsifiants, des coémulsifiants, des épaississants/régulateurs de viscosité/stabilisants, des antioxydants, des hydrotropes (ou polyols), des solides et des charges, des additifs nacrés, des répulsifs pour insectes, des agents autobronzants, des conservateurs, des après-shampooings, des parfums, des matières colorantes, des substances actives cosmétiques, des additifs de soins, des agents surgras et des solvants.

9. Procédé pour diminuer les décolorations d'un textile causées par une formulation d'écran solaire comprenant le stade de procédé
Mise à disposition de la formulation d'écran solaire sous forme d'une composition contenant
A) au moins une substance choisie parmi les desferrioxamines et les produits d'additions d'acide d'une desferrioxamine ou leurs sels, en particulier la desferrioxamine B,
B) au moins une substance de filtre de protection contre de la lumière UV du groupe des substances de filtre de protection contre de la lumière UV organiques, et/ou
sous la forme d'une composition selon au moins l'une des revendications précédentes.

10. Utilisation d'au moins une substance choisie parmi les desferrioxamines et les produits d'addition d'acide d'une desferrioxamine ou leurs sels, en particulier la desferrioxamine B, pour améliorer la capacité de lessivage d'au moins une substance de filtre de protection contre de la lumière UV organique depuis un textile.
